(19) Europäisches Patentamt

European Patent Office

Office européen des brevets

(11) Veröffentlichungsnummer: **0 355 655**
**A2**

(12) **EUROPÄISCHE PATENTANMELDUNG**

(21) Anmeldenummer: **89115010.4**

(22) Anmeldetag: **14.08.89**

(51) Int. Cl.⁴: **G01N 33/84**

Patentanspruch für folgenden Vertragsstaat: ES.

(30) Priorität: **15.08.88 US 232252**

(43) Veröffentlichungstag der Anmeldung:
**28.02.90 Patentblatt 90/09**

(84) Benannte Vertragsstaaten:
**AT BE CH DE ES FR GB IT LI LU NL SE**

(71) Anmelder: **F. HOFFMANN-LA ROCHE AG**
**Postfach 3255**
**CH-4002 Basel(CH)**

(72) Erfinder: **Rosenfeld, Henry Joseph**
**250 Ridgedale Avenue Apartment A4**
**Florham Park, N.J. 07932(US)**

(74) Vertreter: **Lederer, Franz, Dr. et al**
**Patentanwalt Dr. Franz Lederer**
**Lucile-Grahn-Strasse 22**
**D-8000 München 80(DE)**

(54) **Chlorid-Bestimmung.**

(57) Es wird eine Methode zur Messung der Chlorid-Konzentration in Proben von biologischen Flüssigkeiten beschrieben, bei der ein Zweiregenziensystem basierend auf Methansulfonsäure in Kombination mit einem ionischen Netzmittel und Eisen(III)perchlorat verwendet wird.

EP 0 355 655 A2

## Chlorid-Bestimmung

Die vorliegende Erfindung betrifft ein Verfahren zum Messen der Chlorid-Konzentration in einer Probe einer Körperflüssigkeit unter Verwendung eines Zweireagenziensystems basierend auf Methansulfonsäure in Kombination mit einem Netzmittel und Eisen(III)perchlorat.

Die vorliegende Erfindung bezieht sich auf die neuen Reagenzien, die im Verfahren gemäss der Erfindung verwendet werden, sowie auf diagnostische Test-Kits enthaltend diese neuen Reagenzien, welche Test-Kits die Durchführung der Chlorid-Bestimmung entweder manuell oder automatisch ermöglichen.

Chlorid ist das am meisten vorkommende extrazelluläre Anion und ist für die Beibehaltung einer korrekten Hydration, des osmotischen Druckes und eines normalen Anionen-Kationen Gleichgewichtes in Warmblütlern wie Menschen verantwortlich. Niedrige Serumchlorid-Werte können klinisch begleitet sein mit Zuständen wie chronischer Phyelonephritis oder metabolischer Acidose. Erhöhte Serumchlorid-Werte werden bei der Hydration und andern Bedingungen, die den renalen Blutfluss herabsetzen, wie kongestives Herzversagen, beobachtet. Es ist deshalb von Interesse, eine genaue, ökonomische und bequeme Methode zur Bestimmung der Chloridspiegel in Körperflüssigkeiten zu haben.

Es sind mehrere Methoden zur Messung der Chloridionen bekannt. Die meist verbreiteten sind coulometrisch-amperometrische Titrationen, Endpunkttitrationen, Methoden mit spezifischen Elektroden und verschiedene colorimetrische Verfahren. Die klinische Wichtigkeit der Chlorid-Bestimmung verlangt das Testen von Proben im grossen Umfang, wobei Methoden die zur Automation hinführen besonders bevorzugt sind.

Gegenwärtig basieren die am häufigsten benutzten automatischen Methoden zur Bestimmung von Chlorid auf der gutbekannten Quecksilber-Thiocyanat-Reaktion. Dieses Verfahren, welches viele unerwünschte Aspekte zeigt, ist in dem Bereich, in welchem die Serumchlorid-Konzentrationen häufig gefunden werden, nicht strikt linear. Andere Halogenide, wie Bromide und Iodide verursachen erhebliche Interferenzen, und die grossen Mengen von lebensgefährlichen Quecksilber-Salzen, die als Nebenprodukte dieses Verfahrens entstehen, erfordern ein Sicherheitsdispositiv.

Natürlich begann man sich an Nicht-Quecksilber-Methoden zu orientieren, und in 1956 beschrieben West und Coll eine Methode zur Bestimmung von Chlorid in frischem Wasser basierend auf Eisen(III)-perchlorat [Analytical Chemistry, 28: 1834]. Das kritische Reagens, welches Eisen(III)perchlorat und Perchlorsäure enthielt, schaffte eine stark saure Umgebung in der das Chlorid und das Eisen(III)ion einen Komplex bildeten, welcher leicht spektrophotometrisch gemessen werden konnte. Dieses Verfahren erschien nicht praktikabel mit biologischen Proben, welche Protein enthalten und nach Kontakt mit Säure zur Ausfällung neigen. Nichts desto weniger wurde dieses Verfahren zur Messung von Serumchlorid an den Autoanalyzer II adaptiert, welcher vor der analytischen Messung die Probe zur Entfernung der Proteine vorgängig dialysiert [Clinical Chimica Acta 41: 247 (1972)]. Die Proteinausfällung wurde somit als Problem eliminiert.

Eine weitere Entwicklung umfasste die Zugabe hoher Konzentrationen nicht-ionischer Netzmittel zum Eisen(III)perchlorat/perchlorsäure-Reagens zur Eliminierung sowohl der Proteinausfällung wie der Notwendigkeit für Dialyse [Clinical Chemistry, 26: 1874 (1980)].

Die meisten klinischen Proben enthalten eine Vielfalt von verschiedenen Analyten, wobei einige mit der Messung von Chlorid interferieren. Bei Chloridbestimmungen gemäss dieser Methode ist Bilirubin der Hauptstörfaktor. Die Entwicklung eines bichromatischen Blankingverfahrens im Zusammenhang mit dieser Methodik eliminiert dieses Problem [U.S. Patent Nr. 4,278,440]. Es war jedoch notwendig, Absorptionsmessung bei verschiedenen Wellenlängen durchzuführen, was die Verwendung dieses Verfahrens mit automatisierten Methoden erschwierigte.

Die vorliegende Erfindung betrifft ein Verfahren zur Messung der Chlorid-Konzentration in einer Probe einer Körperflüssigkeit enthaltend die Schritte:

(a) Versetzen der Probe mit einem Reagenz enthaltend Methansulfonsäure und ein ionisches Netzmittel (Reagens 1),

(b) Messen der Absorption des Reaktionsgemisches bei 320-380 nm,

(c) Versetzen des Reaktionsgemisches mit einem zweiten Reagens enthaltend Eisen(III)perchlorat, Methansulfonsäure und ein ionisches Netzmittel (Reagens 2) und

(d) Messen der Absorption der Probe bei 320-380 nm, wobei die Differenz in der Absorption mit der Konzentration von Chlorid in der Probe korreliert.

Die vorliegende Erfindung betrifft auch die in der Chloridionen-Bestimmung gemäss vorliegender Erfindung verwendeten Reagenzien, nämlich Reagens 1 und Reagens 2.

Die vorliegende Erfindung betrifft ferner ein diagnostisches Kit zur Bestimmung von Chlorid in einer Probe einer Körperflüssigkeit enthaltend:

    (a) einen Behälter mit Reagens 1

    (b) einen Behälter mit Reagens 2

    (c) einen Behälter mit einem hohen Kontrollwert

    (d) einen Behälter mit einem niedrigen Kontrollwert

    (e) Behälter mit Referenzstandards.

Die Methode gemäss der Erfindung kann zur Messung der Chloridionen-Konzentration in jeder Probe einer Körperflüssigkeit verwendet werden. Einzige Voraussetzung ist, dass, wenn die Probe der Körperflüssigkeit auf Protein basiert (wie Serum), dass dann die Standards und Kontrollen ebenfalls auf Proteinbasis sein müssen und, sofern die Probe auf wässriger Basis (wie Urin) ist, dass die Standards und Kontrollen ihrerseits auf wässriger Basis sein müssen. Die Probe der Körperflüssigkeit ist vorzugsweise Serum oder Urin.

Reagens 1 enthält eine Lösung von ionischem Netzmittel und Methansulfonsäure. Das ionische Netzmittel ist vorzugsweise ein kationisches polyäthoxyliertes tertiäres Stearylamin, z.B. Chemeen 18-50, welches bei Chemax Inc., Greenville, South Carolina, hergestellt wird. Reagens 1 enthält ungefähr von 0,1 bis 7% des kationischen polyäthoxylierten tertiären Stearylamins und von 0,1 bis 1,0 mol/l Methansulfonsäure. Vorzugsweise enthält Reagens 1 30 g/l des kationischen polyäthoxylierten tertiären Stearylamin-Netzmittels Chemeen 18-50 und ungefähr 3,33% Methansulfonsäure (von einer Reinheit >98%), wobei 30 g Netzmittel und 33,3 ml Methansulfonsäure zu 1+000 ml mit entionisiertem Wasser verdünnt werden.

Reagens 2 wird in der gleichen Weise wie Reagens 1 hergestellt und enthält zusätzlich 5 bis 500 g/l Eisen(III)perchlorat. Reagens 2 kann vorzugsweise ungefähr 80 g/l eines "nicht-gelben" Eisen(III)perchlorat enthalten. Reagens 1 und Reagens 2 sollten vor der Verwendung filtriert werden, wobei ein 0,22 Mikronfilter geeignet ist.

Die Messung der Chlorid-Konzentration wird durch Reaktion einer spezifischen Menge einer Probe von Körperflüssigkeit mit einer spezifischen Menge von Reagens 1 durchgeführt, worauf eine erste Absorptionsmessung zur Erfassung des Probenleerwerts erfolgt. Die Mengen von Probe und Reagens hängen von den Mitteln ab, welche zur Messung der Absorption verwendet werden. Wenn z.B. die Bestimmung auf einem Zentrifugalanalysator, wie dem COBAS BIO oder COBAS FARA (Hoffmann-La Roche Inc.) durchgeführt werden, werden vorzugsweise 75 $\mu$l Reagens 1 mit 10 $\mu$l Probe gemischt, wie dies in Beispiel 2 ausgeführt wird. Wenn die Bestimmungen auf einem Zentrifugalanalysator, wie dem COBAS MIRA (Hoffmann-La Roche Inc.) durchgeführt werden, sind die Mengen grösser, und es werden vorzugsweise 100 $\mu$l Reagens 1 mit 15 $\mu$l Probe vermischt, wie dies in Beispiel 3 ausgeführt wird. Falls die Bestimmungen manuell erfolgen, sind die Mengen vom Reagens 1 und Probe notwendigerweise erheblich grösser, und es werden vorzugsweise 750 $\mu$l Reagens 1 mit 100 $\mu$l Probe vermischt. Die Mengen von Probe und Reagens 1 variieren je nach Reaktionsbedingungen, dem zur Messung verwendeten Spektrophotometern und andern Instrumenten sowie andern Variablen. Die Optimierung des Verhältnisses von Probe und Reagens um mit den gewünschten Bestimmungsmethoden übereinzustimmen, liegen im Bereich des allgemeinen Fachwissens.

Nachdem eine geeignete Menge von Probe und Reagens 1 gemischt wurden, folgt eine kurze Inkubation um ein Temperaturgleichgewicht zu erhalten. Die verwendete Methode zur Chlorid-Bestimmung bestimmt das Temperaturgleichgewicht. Für den Fall, dass das Verfahren manuell durchgeführt wird, ist das Gleichgewicht Raumtemperatur. Bei der automatischen Analyse bestimmt der verwendete Automaten-Typ das Temperaturgleichgewicht. Die erste Absorptionsmessung wird bei einer Wellenlänge von 320-380 nm durchgeführt und umfasst den Probenleerwert. Vorzugsweise wird die Absorption bei 340 nm abgelesen.

Die Gesamtchloridbestimmung der unbekannten Probe wird durch Zugabe einer spezifischen Menge von Reagens 2 zum Reaktionsgemisch und Durchführen einer zweiten Absorptionsmessung bestimmt. Die Methode nach welcher das Chlorid gemessen wird bestimmt die Menge von Reagens 2, das zum Reaktionsgemisch zugegeben werden muss. Falls die Bestimmungen z.B. auf einem COBAS BIO oder COBAS FARA durchgeführt werden, wird nach der Probenleerwertbestimmung sofort Reagens 2 zur Reaktionsmischung gegeben und die zweite Absorptionsmessung wird vorgenommen. Vorzugsweise werden ungefähr 75 $\mu$l von Reagens 2 hinzugefügt. Falls die Bestimmungen auf einem COBAS MIRA durchgeführt werden, sind die Mengen von Reagens 2 etwas grösser. Vorzugseise 90 $\mu$l Reagens 2 wird im zweiten Zyklus hinzugefügt. Falls die Bestimmung manuell durchgeführt wird, wird der Probenleerwert durch Messen der Absorption nach Mischen von Reagens 1 mit der Probe bestimmt. Die zweite Absorptionsmessung wird dann in einem zweiten Reagensglas durchgeführt und zwar in einem Reaktionsgemisch, welches nach Mischen von Reagens 1, Reagens 2 und der Probe erhalten wurde. Obwohl die Mengen von Reagens 1 und 2 und Probe variieren können, werden vorzugsweise ungefähr 750 $\mu$l von Reagens 1 mit ungefähr 100 $\mu$l Probe und ungefähr 750 $\mu$l Reagens 2 gemischt.

3

Die Mengen von Reagens 2, welche dem Reaktionsgemisch zugefügt werden, können je nach den Bedingungen der Analyse und der Methode, nach welcher die Chlorid-Konzentration bestimmt wird, sowie andern Variablen variiert werden. Die geeigneten Mengen und Bedingungen können leicht durch einen Fachmann optimiert werden.

Die zweite Absorptionsmessung, welche die gesamte Probenabsorption bildet, wird dann bei 320-380, vorzugsweise 340 nm vorgenommen. Diese zweite Absorptionsmessung sollte innerhalb von 60 Sek. nach Zugabe von Reagens 2 durchgeführt werden. Die Bildung des $[FeCl]^{2+}$-Komplexes ist sehr rasch und ist ungefähr nach 4,5 Sek. zu 98% vollständig und nach 20 Sek. zu 100% vollständig. Bilirubin wird bei 340 nm ebenfalls stark absorbieren. Bilirubin oxidiert jedoch sehr langsam unter den Reaktionsbedingungen der Erfindung. Bilirubin wird als Störfaktor eliminiert, indem man die zweite Absorptionsmessung innerhalb von 60 Sek., vorzugsweise früher, nach Hinzufügen von Reagens 2 durchführt.

Die Chlorid-Konzentration in der unbekannten Probe und in den Kontrollen können dann in bekannter Weise berechnet werden durch Subtrahieren der ersten Absorption des Probenleerwertes von demjenigen der zweiten Absorption der Gesamtprobe.

Die neuen Reagenzien gemäss der Erfindung, nämlich Reagens 1 und Reagens 2 werden, wie im Beispiel 1 beschrieben, hergestellt. Die Verhältnisse der Bestandteile können innerhalb der hier erwähnten Grenzen variieren und können leicht optimiert werden, damit sie mit den individuellen Reaktionsbedingungen übereinstimmen.

Das diagnostische Testkit, welches für die Chlorid-Bestimmung gemäss vorliegender Erfindung verwendet wird, umfasst:

(a) einen Behälter mit Reagens 1
(b) einen Behälter mit Reagens 2
(c) einen Behälter mit einem hohen Kontrollwert
(d) einen Behälter mit einem niedrigen Kontrollwert
(e) Behälter mit Referenzstandards.

Reagens 1 und 2 werden wie hier beschrieben hergestellt. Hohe und niedrige Chlorid-Kontrollen können leicht von käuflichen Quellen erhalten werden oder können nach bekannten Methoden wie Zusammenlegen oder Verdünnen oder Aufstocken der Kontrollen oder Substanzen mit bekannten Chloridwerten erhalten werden. Referenzstandards sind kommerziell erhältlich von verschiedenen Quellen. Die diagnostischen Testkits gemäss vorliegender Erfindung können zur Chlorid-Bestimmung gemäss den automatischen Methoden wie z.B. einem Zentrifugalanalysator, z.B. COBAS BIO, COBAS FARA oder COBAS MIRA oder für manuelle Chlorid-Bestimmungen verwendet werden. Solche Testkits werden unter Einschluss von Reagens 1 und 2 nach den hier angegebenen Methoden und im Speziellen in Uebereinstimmung mit Beispiel 1 hergestellt. Die Kontrollen und Standards, welche verwendet werden können, sind sehr leicht erhältlich und die Chlorid-Bestimmungen unter Verwendung dieser diagnostischen Kits werden nach den hier beschriebenen Methoden insbesondere nach den Beispielen durchgeführt.

Die vorliegende Erfindung wird in den nachfolgenden Beispielen weiterhin beschrieben, welche lediglich zur Illustration dienen.

## Beispiel 1

## Herstellung der Reagenzien

Reagens 1 wird durch Lösen von 30 g des ionischen Chemeen 18-50 (hergestellt von Chemax Inc.) und 33,3 ml Methansulfonsäure (Reinheit >98%) in 1'000 ml entionisiertem Wasser hergestellt. Die Lösung wird dann zur Eliminierung der matten Unklarheit, welche zuerst erscheint, durch ein 0,22 Mikronfilter filtriert.

Reagens 2, welches in ähnlicher Weise wie Reagens 1 hergestellt wird, enthält zusätzlich 80 g/l Eisen-(III) perchlorat. Es sollte "nicht-gelbes" Eisen(III)perchlorat verwendet werden, da die niederen Grade "gelblich" andere Chloridionen und andere Unreinheiten enthalten können. Reagens 2 sollte in der gleichen Weise wie Reagens 1 filtriert werden.

## Beispiel 2

4

## Chlorid-Bestimmung auf dem COBAS BIO oder COBAS FARA

75 μl Reagens 1 wird mit 10 μl Probe gemischt. Es folgt eine kurze Inkubation zur Erreichung des Temperaturgleichgewichtes. Es wird eine erste Absorptionsmessung, welche den Probenleerwert darstellt, bei 340 nm durchgeführt. Darauf werden 75 μl Reagens 2 zum Gemisch zugegeben und eine zweite Absorptionsmessung wird bei 340 nm innerhalb von 60 Sek. durchgeführt. Die Bildung des $[FeCl]^{2+}$-Komplexes ist sehr rasch und üblicherweise nach 20 Sek. vollständig. Bereits nach 4,5 Sek. ist jedoch die Reaktion zu 98% vollständig. Paralell werden Standards auf Proteinbasis bestimmt, welche die Berechnung der Chloridkonzentration in der unbekannten Probe ermöglichen.

## Beispiel 3

## Chlorid-Bestimmung auf dem COBAS MIRA

Die Reagenzien 1 und 2 sind wie in Beispiel 1 beschrieben. 100 μl Reagens 1 wird mit 15 μl Probe gemischt. Nach einer kurzen Inkubation zur Erreichung des Temperaturgleichgewichtes wird die Absorption des Probenleerwerts bei 340 nm während Zyklus 1 gemessen. Während Zyklus 2 werden 90 μl Reagens 2 hinzugegeben und die Absorption der unbekannten Probe wird bei 340 nm gemessen. Falls die unbekannten Proben auf Serum oder Protein basierenden Flüssigkeiten beruhen, muss ein auf Protein basierender Standard verwendet werden. Für andere, nicht auf Protein basierende Proben müssen entsprechende Standards verwendet werden, die nicht auf Protein-Basis beruhen.

## Beispiel 4

## Manuelle Chloridbestimmung

750 μl Reagens 1 wird mit 100 μl der unbekannten Probe in einem Reagensglas gemischt. Nach einer kurzen Zeit wird das Temperaturgleichgewicht erhalten, worauf die Absorption des Probenleerwertes bei 340 nm mit einem Gilford-Spektrophotometer gemessen wird.

750 μl Reagens 1 werden mit 100 μl der unbekannten Probe und 750 μl Reagens 2 in einem Reagenzglas gemischt. Nach einer kurzen Zeit, in der das Temperaturgleichgewicht hergestellt wird, wird eine zweite Absorptionsmessung innerhalb von 60 Sek. bei 340 nm durchgeführt.

Paralell zu den unbekannten Proben werden Standards gemessen und die Chlorid-Konzentration wird aufgrund der Standardkurve nach an sich bekannten Methoden berechnet.

## Beispiel 5

## Vergleich von Chlorid-Bestimmungen in verschiedenen Arten von Proben

In einer Anzahl von Humanserum-Proben werden die Chloridionen nach der COBAS MIRA-Methode und nach der Ionen spezifischen Methode unter Verwendung eines Beckmann E4A- Gerätes bestimmt. Reagenzien in Uebereinstimmung mit Beipiel 1 werden verwendet und die Chlorid-Bestimmungen werden auf dem COBAS MIRA gemäss Beispiel 3 durchgeführt.

Die Ergebnisse dieser Vergleichsstudie werden in der nachfolgenden Tabelle 1 zusammengefasst:

Tabelle 1

| Art der humanen Serumprobe | Chloridkonzentration Beckmann E4A (meq/l) | Chloridkonzentration COBAS MIRA (meq/l) |
|---|---|---|
| normal | 101 | 103 |
| lipämisch | 104 | 105 |
| sehr lipämisch | 104 | 104 |
| normal | 105 | 105 |
| normal | 107 | 107 |
| sehr lipämisch | 108 | 108 |
| hämolysiert | 106 | 106 |
| verdünnt (3:4) | 76 | 76 |
| verdünnt (5:6) | 85 | 87 |
| verdünnt (11:12) | 94 | 94 |
| aufgestockt | 119 | 120 |
| aufgestockt | 128 | 128 |
| aufgestockt | 135 | 137 |

Beispiel 6

Es werden auf dem COBAS MIRA weitere Chlorid-Analysen durchgeführt, bei denen die Proben verschieden lang nach Zugabe von Reagens 2 reagieren konnten. Die Resultate sind in der nachfolgenden Tabelle 2 zusammengefasst und, zeigen wie die Interferenz von Bilirubin durch Verkürzung der Reaktionszeit wesentlich eliminiert werden kann.-

Tabelle 2

| Bilirubin-Konzentration mg/dl | Chlorid-Konzentration in meq/l | | | | |
|---|---|---|---|---|---|
| | Reaktionszeit (Sekunden): | | | | |
| | 25 | 50 | 75 | 100 | 150 |
| keine | 103 | 103 | 103 | 103 | 103 |
| 2 | 103 | 104 | 104 | 104 | 104 |
| 5 | 105 | 107 | 107 | 107 | 107 |
| 10 | 104 | 106 | 108 | 110 | 112 |
| 15 | 105 | 109 | 113 | 115 | 118 |
| 20 | 105 | 110 | 115 | 118 | 121 |

Beispiel 7

Es werden Chlorid-Konzentrationen verschiedener Proben, welche auf dem COBAS MIRA gemäss vorliegender Erfindung bestimmt wurden, mit Chlorid-Konzentrationen, welche nach der Quecksilberthiocyanat-Methode bestimmt wurden, miteinander verglichen. Beide Methoden wurden korreliert mit der Chlorid-Messung unter Verwendung einer Ionen spezifischen Elektrode in einem Beckmann E4A-Gerät.

Die Resultate sind in der folgenden Tabelle 3 zusammengefasst.

Tabelle 3

| Verglichene Funktion | | Erfindungsgemäss | | Methode Quecksilberthiocyanat |
|---|---|---|---|---|
| 1. | Linearität | 0-140 meq/l | | 80-120 meq/l |
| 2. | Korrelation | | | |
| | Steigung | 1,01 | | 0,962 |
| | Abschnitt | +0,12 | | +7,4 |
| | R | 0,993 | | 0,983 |
| | n | 56 | | 51 |
| 3. | Präzision (innerhalb des Verfahrens) | I | II | III |
| | X | 104,6 | 122,1 | 107,6 |
| | S.D. | 1,07 | 1,03 | 2,28 |
| | CV% | 1,0 | 0,84 | 2,1 |
| | n | 20 | 20 | 20 |
| 4. | Stabilität: | 1 Jahr bei 25° C | | |
| 5. | Halogenid-Interferenz | | % Aenderung | |
| | 10mM NaBr | | 0 | +20 |
| | 10mM NaF | | 0 | 0 |
| | 10mM KJ | | 0 | +23 |

Obwohl die Erfindung im Zusammenhang mit bevorzugten Ausführungsformen beschrieben wurde, ist nicht beabsichtigt, den Umfang der Erfindung auf diese individuellen Formen zu limitieren, sondern es ist ganz im Gegenteil so gemeint, dass alternative Modifikationen und Aequivalente vom Geist und Umfang der Erfindung mitumfasst werden, wie sie in den nachfolgenden Ansprüchen beschrieben sind.

## Ansprüche

1. Verfahren zum Messen der Chlorid-Konzentration in einer Probe einer biologischen Flüssigkeit, umfassend die folgenden Schritte:
(a) Versetzen der Probe mit einem Reagens enthaltend Methansulfonsäure und ionisches Netzmittel (Reagenz 1),
b) Messen der Absorption des Reaktionsgemisches bei 320-380 Nanometern,
(c) Versetzen des Reaktionsgemisches mit einem zweiten Reagens enthaltend Eisen (III) Perchlorat, Methansulfonsäure und ein ionisches Netzmittel (Reagens 2),
(d) Messen der Absorption des Reaktionsgemisches bei 320-380 Nanometern,
wobei die Differenz in der Absorption mit der Konzentration vom Chlorid in der Probe korreliert.
2. Verfahren nach Anspruch 1, dadurch gekennzeichnet, dass die Körperflüssigkeit Serum oder Urin ist.
3. Verfahren nach Anspruch 1 oder 2, dadurch gekennzeichnet, dass Reagens 1 ein kationisches polyäthoxyliertes tertiäres Stearylamin-Netzmittel, vorzugsweise von 0,1 bis 7%, und Methansulfonsäure, vorzugsweise von 0,1 bis 1,0 mol/l, umfasst.
4. Verfahren nach Ansprüchen 1 bis 3, dadurch gekennzeichnet, dass Reagens 2 ein kationisches polyäthoxyliertes Stearylamin-Netzmittel, Methansulfonsäure und Eisen(III)perchlorat, vorzugseise von 5 bis 500 g/l umfasst.
5. Verfahren nach Ansprüchen 1 bis 4, dadurch gekennzeichnet, dass die Absorption bei ungefähr 340 Nanometern gemessen wird.
6. Verfahren nach Ansprüchen 1 bis 5, dadurch gekennzeichnet, dass die Chloridbestimmung auf einem

7

Zentrifulgal-Analysator durchgeführt wird.

7. Reagenzien-Zusammensetzung verwendbar für die Chloridbestimmung in Körperflüssigkeiten umfassend Methansulfonsäure und ein ionisches Netzmittel (Reagens 1).

8. Reagenzien-Zusammensetzungen nach Anspruch 7, enthaltend von 0,1 bis 7% eines kationischen polyäthoxylierten tertiären Stearylamin-Netzmittels und 0,1 bis 1,0 mol/l Methansulfonsäure.

9. Reagenzien-Zusammensetzung verwendbar für die Bestimmung von Chlorid in Körperflüssigkeiten umfassend Methanfulfonsäure, ein ionisches Netzmittel und Eisen(III)perchlorat (Reagens 2), vorzugsweise 5 bis 500 g/l.

10. Diagnostisches Kit verwendbar für die Bestimmung von Chlorid-Konzentration in Körperflüssigkeiten umfassend

   (a) einen Behälter mit Reagens 1
   (b) einen Behälter mit Reagens 2
   (c) Behälter mit hohen und niedrigen Chlorid-Kontrollen
   (d) Behälter mit Referenz-Standards.

Patentansprüche für folgende Vertragsstaaten: ES

1. Verfahren zum Messen der Chlorid-Konzentration in einer Probe einer biologischen Flüssigkeit, umfassend die folgenden Schritte:
(a) Versetzen der Probe mit einem Reagens enthaltend Methansulfonsäure und ionisches Netzmittel (Reagenz 1),
(b) Messen der Absorption des Reaktionsgemisches bei 320-380 Nanometern,
(c) Versetzen des Reaktionsgemisches mit einem zweiten Reagens enthaltend Eisen (III) Perchlorat, Methansulfonsäure und ein ionisches Netzmittel (Reagens 2),
(d) Messen der Absorption des Reaktionsgemisches bei 320-380 Nanometern,
wobei die Differenz in der Absorption mit der Konzentration vom Chlorid in der Probe korreliert.

2. Verfahren nach Anspruch 1, dadurch gekennzeichnet, dass die Körperflüssigkeit Serum oder Urin ist.

3. Verfahren nach Anspruch 1 oder 2, dadurch gekennzeichnet, dass Reagens 1 ein kationisches polyäthoxyliertes tertiäres Stearylamin-Netzmittel, vorzugsweise von 0,1 bis 7%, und Methansulfonsäure, vorzugsweise von 0,1 bis 1,0 mol/l, umfasst.

4. Verfahren nach Ansprüchen 1 bis 3, dadurch gekennzeichnet, dass Reagens 2 ein kationisches polyäthoxyliertes Stearylamin-Netzmittel, Methansulfonsäure und Eisen(III)perchlorat, vorzugseise von 5 bis 500 g/l umfasst.

5. Verfahren nach Ansprüchen 1 bis 4, dadurch gekennzeichnet, dass die Absorption bei ungefähr 340 Nanometern gemessen wird.

6. Verfahren nach Ansprüchen 1 bis 5, dadurch gekennzeichnet, dass die Chloridbestimmung auf einem Zentrifulgal-Analysator durchgeführt wird.